# EUROPEAN PATENT APPLICATION

(11) **EP 4 497 818 A1**
(43) Date of publication of application: **29.01.2025**
(21) Application number: 23187451.2
(22) Date of filing: 25.07.2023
(51) Int. Cl.: C12N 1/20, C12N 9/02, C12P 13/08, C12R 1/15

(54) **METHOD FOR THE FERMENTATIVE PRODUCTION OF L-LYSINE USING C. GLUTAMICUM STRAINS EXPRESSING HETEROLOGOUS NICOTINAMIDE NUCLEOTIDE TRANSHYDROGENASE PNTAB**

(71) Applicant: Evonik Operations GmbH, 45128 Essen (DE)
(72) Inventor: RAMOS VERA, Hugo, 33611 Bielefeld (DE); JERRENTRUP, Silke, 33607 Bielefeld (DE); KOTTENHAHN, Patrick, 33602 Bielefeld (DE)
(74) Representative: Evonik Patent Association

(57) **Abstract**

The present invention concerns C. *glutamicum* strains expressing NAD(P)(+) transhydrogenase subunit alpha PntA and NAD(P)(+) transhydrogenase subunit beta PntB of *Corynebacterium urealyticum* and a method for the fermentative production of L-lysine using such *C*. *glutamicum* strains.

## Description

The present invention concerns *Corynebacterium glutamicum* (*C. glutamicum*) strains expressing NAD(P)(+) transhydrogenase subunit alpha PntA and NAD(P)(+) transhydrogenase subunit beta PntB of *Corynebacterium urealyticum* and a method for the fermentative production of L-lysine using such *C**.** glutamicum* strains.

A critical factor in the biotechnological production of L-lysine with *C**.** glutamicum* is the sufficient supply of NADPH. To produce one molecule of L-lysine, four molecules of NADPH are required to drive the necessary reducing steps. NADPH is provided from reactions of the pentose phosphate pathway (PPP) and the citric acid cycle (TCA). For example, Ohnishi et al. showed that mutation of the *gnd* gene in the PPP to alleviate feedback regulation of the enzyme led to increased lysine production (Ohnishi, J., Katahira, R., Mitsuhashi, S., Kakita, S., & Ikeda, M. (2005). A novel gnd mutation leading to increased L-lysine production in Corynebacterium glutamicum. FEMS microbiology letters, 242(2), 265-274).

A further option is the introduction of an NADP-dependent glyceraldehyde 3-phosphate dehydrogenase (GapN) of *Streptococcus mutans* which led to increased NADPH availability and thus increased lysine yields (Takeno, S., Murata, R., Kobayashi, R., Mitsuhashi, S., & Ikeda, M. (2010). Engineering of Corynebacterium glutamicum with an NADPH-generating glycolytic pathway for L-lysine production. Applied and environmental microbiology, 76(21), 7154-7160). If an NADP dependent Gap-Enzyme is used instead of an NAD dependent one, energy conservation in form of ATP generation is omitted in this step, reducing the overall ATP yield per glucose.

Heterologous transhydrogenase activity offers an alternative perspective for sufficient NADPH supply, which is relevant for most amino acid production systems. One example is the use of the membrane-integral nicotinamide nucleotide transhydrogenase PntAB of *Escherichia coli* (*E.coli*), which can use the electrochemical proton gradient across the cytoplasmic membrane to drive the reduction of NADP(+) via the oxidation of NADH. As *C. glutamicum* does not possess such an enzyme, the expression of the heterologous transhydrogenase PntAB gene results in an efficient way to improve the biotechnological production of metabolic compounds (Liu J, Li H, Zhao G, Caiyin Q, Qiao J. Redox cofactor engineering in industrial microorganisms: strategies, recent applications and future directions. J Ind Microbiol Biotechnol. 2018;45(5):313-327). Since the native reactions providing NADPH (*zwf, gnd, icd*) are associated with carbon loss in the form of CO₂ evolution, and GapN has a higher strain on energy conservation, use of PntAB is a preferable route in terms of ATP, carbon yield and environmental impact.

There are PntAB variants from different organisms available. The PntAB variant from *E. coli* is well researched and characterized. Further examples of organisms harbouring *pntAB* variants are *Oenococcus oeni, Synechocystis sp.* and *Lactobacillus buchneri* (Liu S, Skory C, Liang X, Mills D, Qureshi N. Increased ethanol tolerance associated with the pntAB locus of Oenococcus oeni and Lactobacillus buchneri. J Ind Microbiol Biotechnol. 2019;46(11):1547-1556). The *pntAB* variant from *Corynebacterium urealyticum* (*C. urealyticum*), a closer relative to *C. glutamicum,* has to date not been investigated in such detail.

Several studies describe the heterologous expression of membrane-integral nicotinamide nucleotide transhydrogenase PntAB of *E. coli* to improve L-amino acids production by *C. glutamicum* e.g., L-lysine, L-arginine, L-valine, L-methionine (Kabus A, Georgi T, Wendisch VF, Bott M. Expression of the Escherichia coli pntAB genes encoding a membrane-bound transhydrogenase in Corynebacterium glutamicum improves L-lysine formation. Appl Microbiol Biotechnol. 2007;75(1):47-53). In addition, succinate production is also described to be improved by expression of heterologous transhydrogenase *pntAB* gene of *E. coli.* For example, Zhan et al. provide their study data that expression of *pntAB* increased L-arginine titer from 41.5 to 56.6 g L⁻¹, suggesting its critical effect on NADPH supply and L-arginine production (Zhan M., Kan B., Dong J., Han R., Ni Y. Metabolic engineering of Corynebacterium glutamicum for improved L-arginine synthesis by enhancing NADPH supply. J. Ind. Microbiol. Biotechnol. 2019; 46(1):45-54).

Wang (Wang C, Zhou Z, Cai H, Chen Z, Xu H. Redirecting carbon flux through pgi-deficient and heterologous transhydrogenase toward efficient succinate production in Corynebacterium glutamicum. J Ind Microbiol Biotechnol. 2017;44(7):1115-1126) showed a positive impact of *pntAB* from *E. coli* on succinate production with *C. glutamicum.* Moreover, Bartek et al. demonstrated for L-valine that this bypass leads to a significant increase of product yield due to a concurrent reduction in carbon dioxide formation via the PPP (Bartek T., Blombach B., Lang S., Eikmanns B., Wiechert W., Oldiges M., Nöh K., and Noack S. Comparative 13C metabolic flux analysis of pyruvate dehydrogenase complex-deficient, L-valine-producing Corynebacterium glutamicum. Appl Environ Microbiol. 2011; 77(18): 6644-6652). Liu provided data evaluating the differential impact of TCA, PPP and *pntAB* from E. *coli* on NADPH availability and L-methionine production with *C. glutamicum* (Liu, B.; Sun, X.; Liu, Y.; Yang, M.; Wang, L.; Li, Y.; Wang, J. Increased NADPH Supply Enhances Glycolysis Metabolic Flux and L-methionine Production in Corynebacterium glutamicum. Foods 2022, 11, 1031. https://doi.org/ 10.3390/foods11071031).

Wittmann (Wittmann, C., & Heinzle, E. (2002). Genealogy profiling through strain improvement by using metabolic network analysis: metabolic flux genealogy of several generations of lysine-producing corynebacteria. Applied and environmental microbiology, 68(12), 5843-5859) investigated the metabolic progress of several generations of improved L-lysine producing strains of *C. glutamicum* and found that due to increased lysine production, the NADPH requirement increased from 109 to 172%, while the NADPH pool remained constant between 185 and 196%, expecting the NADPH pool to become limiting in an industrial setting. Additionally, NADPH supply is known to be a limiting factor for lysine production at lysine yields ≥55% (m/m) or ≥45% (g/g), (Kjeldsen, K. R., & Nielsen, J. (2009). In silico genome-scale reconstruction and validation of the Corynebacterium glutamicum metabolic network. Biotechnology and bioengineering, 102(2), 583-597).

To further improve the NADPH availability in highly efficient lysine production strains of *C. glutamicum* according to the present invention, a *C. glutamicum* strain is provided, comprising overexpressed *asd, aspB, dapA, dapB, lysA, lysC* and *ddh* genes coding for enzymes having the function of aspartate-semialdehyde dehydrogenase, aspartate aminotransferase, dihydrodipicolinate synthase, dihydrodipicolinate reductase, diaminopimelate decarboxylase, aspartatokinase and diaminopimelate dehydrogenase, respectively, and comprising a gene coding for the NAD(P)(+) transhydrogenase subunit alpha PntA comprising an amino acid sequence with an identity of at least 80 % to the amino acid sequences according to SEQ ID NO: 7 and a gene coding for the NAD(P)(+) transhydrogenase subunit beta PntB comprising an amino acid sequence with an identity of at least 80 % to the amino acid sequences according to SEQ ID NO: 8.

The NAD(P)(+) transhydrogenase subunit alpha PntA with the sequence according to SEQ ID NO: 7 and the NAD(P)(+) transhydrogenase subunit beta PntB according to SEQ ID NO: 8 are derived from *Corynebacterium urealyticum.*

When comparing the amino acid sequences of the polypeptides derived from *E. coli* (SEQ ID NO: 3) and *Corynebacterium urealyticum* (SEQ ID NO: 7) having the function of NAD(P)(+) transhydrogenase subunit alpha PntA the identity was found to be 57 %. Comparing the amino acid sequences of the polypeptides derived from *E. coli* (SEQ ID NO: 4) and *Corynebacterium urealyticum* (SEQ ID NO: 8) having the function of NAD(P)(+) transhydrogenase subunit beta PntB the identity was found to be 66 %.

The C. *glutamicum* strain according to the present invention comprises a gene coding for a NAD(P)(+) transhydrogenase subunit alpha PntA that comprises an amino acid sequence preferably with an identity of at least 80 %, more preferably of at least 90 %, most preferably of at least 95 % to the amino acid sequences according to SEQ ID NO: 7 and comprises a gene coding for a NAD(P)(+) transhydrogenase subunit beta PntB that comprises preferably an amino acid sequence with an identity of at least 80 %, more preferably of at least 90 % identity, most preferably of at least 95 % identity to the amino acid sequences according to SEQ ID NO: 8.

In a particular embodiment of the present invention the highly efficient L-lysine production strains of *C. glutamicum* strain according to the present invention comprises overexpressed *asd, aspB, dapA, dapB, lysA, lysC* and *ddh* genes coding for enzymes having the function of aspartate-semialdehyde dehydrogenase, aspartate aminotransferase, dihydrodipicolinate synthase, dihydrodipicolinate reductase, diaminopimelate decarboxylase, aspartokinase and diaminopimelate dehydrogenase, respectively, and comprises a gene coding for the NAD(P)(+) transhydrogenase subunit alpha PntA comprising the polynucleotide sequence according to SEQ ID NO: 5 and a gene coding for the NAD(P)(+) transhydrogenase subunit beta PntB comprising the polynucleotide sequence according to SEQ ID NO: 6.

Up to now, there is no information about heterologous expression of membrane-integral nicotinamide nucleotide transhydrogenase PntAB of *Corynebacterium urealyticum* to improve L-lysine production by *C. glutamicum.* Heterologous expression of a *pntAB* gene from *Corynebacterium urealyticum* in highly efficient L-lysine production strains of *C. glutamicum* as defined according to the present invention showed a more beneficial effect on L-lysine production compared to *pntAB* gene of *E. coli.* In less efficient production strains, this effect is not observed.

Overexpression of a gene can be achieved by increasing the copy number of the gene and/or by an enhancement of regulatory factors, e.g. by functionally linking the gene with a strong promoter and/or by enhancing the ribosomal binding site and/or by codon usage optimization of the start codon or of up to the whole gene. The enhancement of such regulatory factors which positively influence gene expression can, for example, be achieved by modifying the promoter sequence upstream of the structural gene in order to increase the effectiveness of the promoter or by completely replacing said promoter with a more effective or a so-called strong promoter. Promoters are located upstream of the gene. A promoter is a DNA sequence consisting of about 40 to 50 base pairs and which constitutes the binding site for an RNA polymerase holoenzyme and the transcriptional start point, whereby the strength of expression of the controlled polynucleotide or gene can be influenced. Generally, it is possible to achieve an overexpression or an increase in the expression of genes in bacteria by selecting strong promoters, for example by replacing the original promoter with strong, native (originally assigned to other genes) promoters or by modifying certain regions of a given, native promoter (for example its so-called -10 and -35 regions) towards a consensus sequence, e.g. as taught by M. Patek et al. (Microbial Biotechnology 6 (2013), 103-117) for *C. glutamicum.* An example for a "strong" promoter is the superoxide dismutase (*sod*) promoter ("Psod"; Z. Wang et al., Eng. Life Sci. 2015, 15, 73-82). A "functional linkage" is understood to mean the sequential arrangement of a promoter with a gene, which leads to a transcription of the gene.

The *Corynebacterium glutamicum* strain according to the present invention is preferably a L-lysine excreting strain. L-lysine excreting strains of the species *Corynebacterium glutamicum* typically contain a polynucleotide coding for a feedback resistant aspartokinase polypeptide variant. A feedback resistant aspartokinase polypeptide variant means an aspartokinase which is less sensitive, or desensitized resp., to inhibition by mixtures of L-lysine and L-threonine, e.g. 10 mM each, or mixtures of the L-lysine analogue S-(2-aminoethyl)-L-cysteine and L-threonine, e.g. 50 mM S-(2-aminoethyl)-L-cysteine and 10 mM L-threonine, when compared to the wild form of the enzyme, which is contained in wild type strains such as ATCC13032, ATCC14067 and ATCC13869. The EC number for aspartokinase is EC 2.7.2.4. Descriptions of polynucleotides of *Corynebacterium glutamicum* encoding a feedback resistant aspartokinase polypeptide variant are given for example in US5688671, US6844176 and US6893848. A summarizing list can be found inter alia in US20090311758A1. The symbol used in the art for a gene coding for an aspartokinase polypeptide is *lysC.* In case the gene codes for a feedback resistant polypeptide variant, the art typically uses symbols like *lysC^{fbr}* with fbr indicating feedback resistance.

The *C. glutamicum strain* according to the present invention further comprises at least one copy of a gene *lysC* coding for a feedback resistant aspartokinase polypeptide variant.

Preferably, the feedback resistant aspartokinase polypeptide variant comprises the amino acid sequence according to SEQ ID NO: 12. The amino acid sequence according to SEQ ID NO: 12 differs from the amino acid sequence of the wild type aspartokinase polypeptide (in ATCC 13032) in that the amino acid threonine (Thr) at position 311 is replaced by the amino acid isoleucine (Ile).

The *C. glutamicum* strain according to the present invention may comprise in its chromosome a polynucleotide encoding an amino acid sequence of a polypeptide having the function of a NAD(P)(+) transhydrogenase subunit alpha PntA comprising SEQ ID NO: 5 and a polynucleotide encoding an amino acid sequence of a polypeptide having the function of a NAD(P)(+) transhydrogenase subunit beta PntB comprising to SEQ ID NO: 6.

The *C. glutamicum* strain of the present invention may comprise in its chromosome a polynucleotide encoding an amino acid sequence of a NAD(P)(+) transhydrogenase subunit alpha PntA comprising an amino acid sequence according to SEQ ID NO: 7 and a polynucleotide encoding an amino acid sequence of a NAD(P)(+) transhydrogenase subunit beta PntB comprising an amino acid sequences according to SEQ ID NO: 8.

It was found that the *C. glutamicum* strain modified according to the invention excreted L-lysine, into a suitable medium under suitable fermentation conditions in an increased manner with respect to e.g. product yield (in g I-lysine/l medium or g L-lysine/g carbon source) as compared to the unmodified bacterium.

Therefore, the present invention also concerns a method for the fermentative production of L-lysine comprising the steps of cultivating the *C. glutamicum* strain of the present invention and accumulating L-lysine in the medium to form an L-lysine containing fermentation broth.

The term L-lysine, where mentioned herein, in particular in the context of product formation, also comprises their ionic forms and salts, for example L-lysine mono hydrochloride or L-lysine sulfate.

The method according to the present invention may further comprise manufacturing an L-lysine containing product from said fermentation broth or isolating L-lysine from the L-lysine containing fermentation broth.

A fermentation broth means a medium in which a *Corynebacterium glutamicum* of the invention has been cultivated for a certain time and under certain conditions.

A suitable medium used for the production of L-lysine by a fermentative process contains a carbon source, a nitrogen source, a phosphorus source, inorganic ions and other organic compounds as required.

Suitable carbon sources include glucose, fructose, sucrose as well as the corresponding raw materials like starch hydrolysate, molasse or high fructose corn syrup.

As nitrogen source organic nitrogen-containing compounds such as peptones, meat extract, soybean hydrolysates or urea, or inorganic compounds such as ammonium sulphate, ammonium chloride, ammonium phosphate, ammonium carbonate, ammonium nitrate, ammonium gas or aqueous ammonia can be used.

As phosphorus source, phosphoric acid, potassium dihydrogen phosphate or dipotassium hydrogen phosphate or the corresponding sodium-containing salts can be used.

Inorganic ions like potassium, sodium, magnesium, calcium, iron and further trace elements etc. are supplied as salts of sulfuric acid, phosphoric acid or hydrochloric acid.

Other organic compounds essentially means growth factors like vitamins e. g. thiamine or biotin or L-amino acids e. g. L-homoserine.

The media components may be added to the culture in form of a single batch or be fed in during the cultivation in a suitable manner.

During the fermentative process the pH of the culture can be controlled by employing basic compounds such as sodium hydroxide, potassium hydroxide, ammonia or aqueous ammonia, or acidic compounds such as phosphoric acid or sulphuric acid in a suitable manner. The pH is generally adjusted to a value of from 6.0 to 8.5, preferably 6.5 to 8.0. To control foaming, it is possible to employ antifoam agents such as, for example, fatty acid polyglycol esters. To maintain the stability of plasmids, it is possible to add to the medium suitable selective substances such as, for example, antibiotics. The fermentative process is preferably carried out under aerobic conditions. In order to maintain these conditions, oxygen or oxygen-containing gas mixtures such as, for example air are introduced into the culture. The fermentative process is carried out, where appropriate, at elevated pressure, for example at an elevated pressure of 0.03 to 0.2 MPa. The temperature of the culture is normally from 25 °C to 40 °C, preferably from 30 °C to 37 °C. In a discontinuous process, the cultivation is continued until an amount of the L-lysine sufficient for being recovered has been formed. The cultivation is then completed. This aim is normally achieved within 10 hours to 160 hours. In continuous processes, longer cultivation times are possible.

Examples of suitable media and culture conditions can be found inter alia in L. Eggeling and M. Bott (Handbook of Corynebacterium glutamicum, CRC Press, 2005) and the patent documents US5770409, US5990350, US 5275940, US5763230 and US6025169.

The fermentation broth is removed from the culture vessel or fermentation tank, collected where appropriate, and used for providing a product containing the L-lysine, in liquid or solid form. In the simplest case, the L-lysine -containing fermentation broth itself, which has been removed from the fermentation tank, constitutes the recovered product.

The fermentation broth can subsequently be subjected to partial to complete or virtually complete removal of the water, partial to complete or virtually complete removal of the biomass, the latter being optionally inactivated before removal, partial to complete or virtually complete removal of the organic by-products formed during the fermentative process, and partial to complete or virtually complete removal of the residual components of the medium employed or of the residual input materials which have not been consumed in the fermentative process.

Removal of water can be achieved inter alia by evaporation, using e.g. a falling film evaporator, by reverse osmosis or nanofiltration. The concentrates thus obtained can be further worked up by spray drying or spray granulation. It is likewise possible to dry the fermentation broth directly using spray drying or spray granulation.

Removal of the biomass can be achieved inter alia by centrifugation, filtration or decantation or a combination thereof.

Removal of the organic by-products or removal of residual components of the medium can be achieved inter alia by chromatography, e.g. ion exchange chromatography, treatment with activated carbon or crystallization. In case the organic by-products or residual components of the medium are present in the fermentation broth as solids they can also be removed by inter alia by centrifugation, filtration or decantation or a combination thereof.

General instructions on separation, purification and granulation methods can be found inter alia in the book of R. Ghosh "Principles of Bioseperation Engineering" (World Scientific Publishing, 2006), the book of F. J. Dechow "Seperation and Purification Techniques in Biotechnology" (Noyes Publications, 1989), the article "Bioseparation" of Shaeiwitz et al. (Ullmann's Encyclopedia of Industrial Chemistry, Wiley-VCH, 2012) and the book of P. Serno et al. "Granulieren" (Editio Cantor Verlag, 2007).

A downstream processing scheme for L-lysine products can be found in the article "L-lysine Production" of R. Kelle et al. (L. Eggeling and M. Bott (Handbook of Corynebacterium glutamicum, CRC Press, 2005)). US5279744 teaches the manufacturing of a purified L-lysine product by ion exchange chromatography. US5431933 teaches the manufacturing of dry L-amino acid products, e. g. an L-lysine product, containing most of the constituents of the fermentation broth.

Thus, a concentration or purification of the L-lysine is achieved and a product having the desired content of said L-lysine is provided.

Finally, L-lysine may be isolated from the culture broth and crystallized in form of a salt, preferably in form of the hydrochloric acid salt of L-lysine.

Analysis of L-lysine to determine its concentration at one or more time(s) during the fermentation can take place by separating the L-lysine by means of ion exchange chromatography, preferably cation exchange chromatography, with subsequent post-column derivatization using ninhydrin, as described in Spackman et al. (Analytical Chemistry 30: 1190-1206 (1958)). It is also possible to employ ortho-phthalaldehyde rather than ninhydrin for post-column derivatization. An overview article on ion exchange chromatography can be found in Pickering (LC.GC (Magazine of Chromatographic Science 7(6):484-487 (1989)). It is likewise possible to carry out a pre-column derivatization, for example using ortho-phthalaldehyde or phenyl isothiocyanate, and to fractionate the resulting amino acid derivates by reversed-phase chromatography (RP), preferably in the form of high-performance liquid chromatography (HPLC). A method of this type is described, for example, in Lindroth et al. (Analytical Chemistry 51:1167-1174 (1979)). Detection is carried out photometrically (absorption, fluorescence). A review regarding amino acid analysis can be found inter alia in the textbook "Bioanalytik" by Lottspeich and Zorbas (Spektrum Akademischer Verlag, Heidelberg, Germany 1998).

### Short description of the sequences

SEQ ID NO: 1 DNA sequence of the *pntA* gene derived from *Escherichia coli* encoding the pyridine nucleotide transhydrogenase subunit alpha, *pntA:* NCBI-GenelD: 946628.
SEQ ID NO: 2 DNA sequence of the *pntB* gene derived from *Escherichia coli* encoding the pyridine nucleotide transhydrogenase subunit beta, *pntB*: NCBI-GeneID: 946144.
SEQ ID NO: 3 PntA polypeptide sequence derived from *Escherichia coli,* NCBI Reference Sequence: PntA: NP_416120.1.
SEQ ID NO: 4 PntB polypeptide sequence derived from *Escherichia coli,* NCBI Reference Sequence: PntB: NP_416119.1.
SEQ ID NO: 5 DNA sequence of the *pntA* gene derived from *Corynebacterium urealyticum* encoding the NAD(P)(+) transhydrogenase subunit alpha, NCBI Reference Sequence: *pntA:* CKV82_RS04765.
SEQ ID NO: 6 DNA sequence of the *pntB* gene derived from *Corynebacterium urealyticum* encoding the NAD(P)(+) transhydrogenase subunit beta, NCBI Reference Sequence: *pntB:* CKV82_RS04770.
SEQ ID NO: 7 PntA polypeptide sequence derived from *Corynebacterium urealyticum,* NCBI Reference Sequence: PntA: WP_012360758.1.
SEQ ID NO: 8 PntB polypeptide sequence derived from *Corynebacterium urealyticum,* NCBI Reference Sequence: PntB: WP_012360759.1.
SEQ ID NO: 9 *pntAB_ec* polynucleotide carrying intergenic sequences derived from *Corynebacterium glutamicum,* promotor sj, *pntA* and *pntB* genes derived from *Escherichia coli.*
SEQ ID NO: 10 *pntAB_cu* polynucleotide carrying intergenic sequences derived from *Corynebacterium glutamicum,* promotor sj, *pntA* and *pntB* genes derived from *Corynebacterium urealyticum.*
SEQ ID NO: 11 DNA sequence of the *lysC* gene encoding the feedback resistant aspartokinase polypeptide variant LysC_T311I.
SEQ ID NO: 12 polypeptide sequence of the feedback resistant aspartokinase polypeptide variant LysC_T311I.

### EXPERIMENTAL SECTION

### A) MATERIALS and METHODS

The molecular biology kits, primers and chemicals used and some details of the methods applied are briefly described herewith.

### 1. Antibiotics and chemicals

a. Kanamycin: Kanamycin solution from *Streptomyces kanamyceticus* from Sigma Aldrich (St. Louis, USA, Cat. no. K0254).
b. Nalidixic acid: Nalidixic acid sodium salt from Sigma Aldrich (St. Louis, USA, Cat. no. N4382).
c. If not stated otherwise, all chemicals were purchased analytically pure from Merck (Darmstadt, Germany), Sigma Aldrich (St. Louis, USA) or Carl-Roth (Karlsruhe, Germany).

### 2. Cultivation

If not stated otherwise, all cultivation / incubation procedures were performed as follows herewith:
a. LB broth (MILLER) from Merck (Darmstadt, Germany; Cat. no. 110285) was used to cultivate *E. coli* strains in liquid medium. The liquid cultures (10 ml liquid medium per 100 ml Erlenmeyer flask with 3 baffles) were incubated in the Infors HT Multitron standard incubator shaker from Infors GmbH (Einsbach, Germany) at 37°C and 200 rpm.
b. LB agar (MILLER) from Merck (Darmstadt, Germany Cat. no. 110283) was used for cultivation of *E. coli* strains on agar plates. The agar plates were incubated at 37°C in an INCU-Line^{®} mini-incubator from VWR (Radnor, USA).
c. Brain heart infusion broth (BHI) from Merck (Darmstadt, Germany; Cat. no. 110493) was used to cultivate *C. glutamicum* strains in liquid medium. The liquid cultures (10 ml liquid medium per 100 ml Erlenmeyer flask with 3 baffles) were incubated in the Infors HT Multitron standard incubator shaker from Infors GmbH (Einsbach, Germany) at 33°C and 200 rpm.
d. Brain heart agar (BHI-agar) from Merck (Darmstadt, Germany; Cat. no. 113825) was used for cultivation of *C. glutamicum* strains on agar plates. The agar plates were incubated at 33°C in an incubator from Heraeus Instruments with Kelvitron^{®} temperature controller (Hanau, Germany).

### 3. Determining optical density

a. The optical density of bacterial suspensions in shake flask cultures was determined at 600 nm (OD600) using the BioPhotometerfrom Eppendorf AG (Hamburg, Germany).
b. The optical density of bacterial suspensions produced in the Wouter Duetz (WDS) micro fermentation system (24-Well Plates) was determined at 660 nm (OD660) with the GENios^{™} plate reader from Tecan Group AG (Männedorf, Switzerland).

### 4. Centrifugation

a. Benchtop centrifuge for reaction tubes with a volume up to 2 ml Bacterial suspensions with a maximum volume of 2 ml were caused to sediment using 1 ml or 2 ml reaction tubes (e.g., Eppendorf Tubes^{®} 3810X) using an Eppendorf 5417 R centrifuge (5 min at 13.000 rpm).
b. Benchtop centrifuge for tubes with a volume up to 50 ml Bacterial suspensions with a maximum volume of 50 ml were caused to sediment using 15 ml or 50 ml centrifuge tubes (e.g. Falcon^{™} 50 ml Conical Centrifuge Tubes) using an Eppendorf 5810 R centrifuge for 10 min at 4.000 rpm.

### 5. PCR

Integration of desired DNA fragments into the chromosome of *C. glutamicum* was detected by PCR amplification and following Sanger sequencing of the PCR product.
a. Primers
The oligonucleotides used were synthesized by eurofins genomics GmbH (Ebersberg, Germany).
b. Template
As PCR template the total DNA contained in a colony was used. It was prepared by taking cell material with a toothpick from a colony on an agar plate and placing the cell material directly into the PCR reaction tube. The cell material was heated for 10 sec with 800 W in a microwave oven type Mikrowave & Grill from SEVERIN Elektrogeräte GmbH (Sundern, Germany) and then the PCR reagents were added to the template in the PCR reaction tube.
b. Reaction Mix
The PCR reaction was carried out using SapphireAmp^{®} Fast PCR Master Mix from Takara Bio Inc. (Shiga, Japan). The reaction mixture was prepared according to the manufacturer's information. Therefore 25 µl of the SapphireAmp^{®} Fast PCR Master Mix (2x) was mixed with 0.5 µl of each of the PCR-Primers [100 pmol/µl] and 24 µl H₂O to get in total 50 µl reaction volume.

**Table 1: Thermocycling conditions for PCR**

| PCR-program | | | |
|---|---|---|---|
| Step | Time [sec] | T [°C] | Description |
| 1 | 60 | 94 | Denaturation |
| | | | Amplification |
| 2 | 05 | 98 | Denaturation step |
| 3 | 05 | 55 | Annealing step |
| 4 | 10 sec/kb | 72 | Elongation step |
| | | | Repeat step 2 to 4: 30 x |

c. PCR Cycler
The reactions were carried out in a Mastercycler^{®} of Eppendorf (Hamburg, Germany).

### 6. Chemical transformation of E. coli

*E. coli* K-12 strain S17-1 was used as donor for conjugational transfer of plasmids based on pK18mobsacB from *E. coli* to *C. glutamicum.* Strain S17-1 is described by Simon, R. et al. (Bio/Technology 1, 784-794, 1983). It is available from the American Type Culture Collection under the access number ATCC47055.

Chemically competent E. *coli* S17-1 cells were made as follows: A preculture of 10 ml LB medium (10 ml liquid medium per 100 ml Erlenmeyer flask with 3 baffles) was inoculated with 100 µl bacterial suspension of strain S17-1 and the culture was incubated overnight for about 18 h at 37°C and 250 rpm. The main culture (70 ml LB contained in a 250 ml Erlenmeyer flask with 3 baffles) was inoculated with 300 µl of the preculture and incubated up to an OD600 of 0.5-0.8 at 37°C. The culture was centrifuged for 6 min at 4°C and 4000 rpm and the supernatant was discarded. The cell pellet was resuspended in 20 ml sterile, ice-cold 50 mM CaCl₂ solution and incubated on ice for 30 min. After another centrifugation step, the pellet was resuspended in 5 ml ice-cold 50 mM CaCl₂ solution and the suspension incubated on ice for 30 min. The cell suspension was then adjusted to a final concentration of 20 % glycerol (v/v) with 85 % (v/v) sterile ice-cold glycerol. The suspension was divided into 50 µl aliquots and stored at -80°C.

To transform S17-1 cells, the protocol according to Tang et al. (Nucleic Acids Res. 22(14), 2857-2858, 1994) with a heat shock of 45 sec. was used.

### 7. Conjugation of C. glutamicum

The pK18mobsacB plasmid system described by Schäfer et al. (Gene 145, 69 - 73, 1994) was used to integrate desired DNA fragments into the chromosome of *C. glutamicum.* A modified conjugation method of Schäfer et al. (Journal of Bacteriology 172, 1663 - 1666, 1990) was used to transfer the respective plasmid into the desired *C. glutamicum* recipient strain.

Liquid cultures of the *C. glutamicum* strains were carried out in BHI medium at 33°C. The heat shock was carried out at 48.5°C for 9 min. Transconjugants were selected by plating the conjugation batch on EM8 agar Table 2), which was supplemented with 25 mg/l kanamycin and 50 mg/l nalidixic acid. The EM8 agar plates were incubated for 72 h at 33°C.

**Table 2: Composition of the EM8 agar**

| Components | Concentration (g/l) |
|---|---|
| Glucose (sterile filtered) | 23 |
| CSL (corn steep liquor; Roquette; solid content 48±2 % w/w) | 30 |
| Peptone from soymeal (Merck, Germany) | 40 |
| (NH₄)₂SO₄ | 8 |
| Urea | 3 |
| KH₂PO₄ | 4 |
| MgSO₄ · 7 H₂O | 0.5 |
| FeSO₄ · 7 H₂O | 0.01 |
| CuSO₄ · 5 H₂O | 0.001 |
| ZnSO₄ · 7 H₂O | 0.01 |
| Calcium pantothenate, D(+) | 0.01 |
| Thiamine | 0.001 |
| Inositol | 0.1 |
| Nicotinic acid | 0.001 |
| Biotin (sterile filtered) | 0.005 |
| CaCO₃ (autoclaved separately) | 1.6 |
| Agar-Agar (Merck, Germany) | 14 |

Sterile toothpicks were used to transfer the transconjugants onto BHI agar, which was supplemented with 25 mg/l kanamycin and 50 mg/l nalidixic acid. The agar plates were incubated for 20 h at 33°C. The cultures of the respective transconjugants produced in this manner were then propagated further for 24 h at 33°C in 10 ml BHI medium contained in 100 ml Erlenmeyer flasks with 3 baffles. An aliquot was taken from the liquid culture suitably diluted and plated (typically 100 to 200 µl) on BHI agar which was supplemented with 10% saccharose. The agar plates were incubated for 48 h at 33°C. The colonies growing on the saccharose containing agar plates were then examined for the phenotype kanamycin sensitivity. To do so a toothpick was used to remove cell material from the colony and to transfer it onto BHI agar containing 25 mg/l kanamycin and onto BHI agar containing 10% saccharose. The agar plates were incubated for 60 h at 33°C. Clones that proved to be sensitive to kanamycin and resistant to saccharose were examined for integration of the desired DNA fragment by means of PCR and following sequencing of the PCR product.

### 8. Glycerol stocks of E. coli and C. glutamicum strains

For long time storage of *E*. *coli-* and *C*. *glutamicum* strains glycerol stocks were prepared. Selected *E. coli* clones were cultivated in 10 ml LB medium supplemented with 2 g/l glucose. Selected *C. glutamicum* clones were cultivated in two-fold concentrated BHI medium supplemented with 2 g/l glucose. Cultures of plasmid containing *E. coli* strains were supplemented with 50 mg/l kanamycin. Cultures of plasmid containing *C. glutamicum* strains were supplemented with 25 mg/l kanamycin. The medium was contained in 100 ml Erlenmeyer flasks with 3 baffles. It was inoculated with a loop of cells taken from a colony and the culture incubated for about 18 h at 37°C and 200 rpm in the case of *E. coli* and 33°C and 200 rpm in the case of *C. glutamicum.* After said incubation period 1.2 ml 85% (v/v) sterile glycerol were added to the culture. The obtained glycerol containing cell suspension was then aliquoted in 2 ml portions and stored at -80°C.

### 9. Cultivation system according to Wouter Duetz (WDS)

The millilitre-scale cultivation system according to Duetz (Trends Microbiol. 2007; 15(10):469-75) was used to investigate the performance of the *C. glutamicum* strains constructed. For this purpose, 24-deepwell microplates (24 well WDS plates) from EnzyScreen BV (Heemstede, Netherlands; Cat. no. CR1424), filled with 2.5 ml medium were used.

Precultures of the strains were done in 10 ml two-fold concentrated BHI medium. The medium was contained in a 100 ml Erlenmeyer flask with 3 baffles. It was inoculated with 100 µl of a glycerol stock culture and the culture incubated for 24 h at 33°C and 200 rpm.

After said incubation period the optical densities OD600 of the precultures were determined.

The main cultures were done by inoculating the 2.5 ml medium containing wells of the 24 Well WDS-Plate with an aliquot of the preculture to give an optical density OD600 of 0.1.

As medium for the main culture SK medium was used. The composition of the SK medium is shown in table 3.

**Table 3: Composition of SK medium.**

| Components | Concentration (g/kg) |
|---|---|
| CSL (corn steep liquor; Roquette; solid content 50 % w/w) | 7.5 |
| MOPS (3-(N-Morpholino) propanesulfonic acid) | 40 |
| (NH₄)₂SO₄ | 25 |
| KH₂PO₄ | 0.1 |
| MgSO₄ · 7 H₂O | 1 |
| CaCl₂ · 2 H₂O | 0.01 |
| FeSO₄ · 7 H₂O | 0.01 |
| MnSO₄ H₂O | 0.005 |
| Biotin (sterile filtered) | 0.0003 |
| Thiamine HCl | 0.0002 |
| Glucose (sterile filtered) | 20.0 |
| NaHCO₃ | 8.4 |
| adjust the pH to 7 with NH₃ | |

These main cultures were incubated for approximately 45 h at 33 °C and 300 rpm in an Infors HT Multitron standard incubator shaker from Infors GmbH (Bottmingen, Switzerland) until complete consumption of glucose.

The glucose concentration in the suspension was analyzed with the blood glucose-meter OneTouch Vita^{®} from LifeScan (Johnson & Johnson Medical GmbH, Neuss, Germany).

After cultivation the culture suspensions were transferred to a deep well microplate. A part of the culture suspension was suitably diluted to measure the OD660. Another part of the culture was centrifuged and the concentration of L-amino acids, in particular L-lysine, and residual glucose were analyzed in the supernatant.

### 10. Amino acid analyser

The concentration of L-lysine and other L-amino acids, e.g. L-valine, in the culture supernatants was determined by ion exchange chromatography using a SYKAM S433 amino acid analyser from SYKAM Vertriebs GmbH (Fürstenfeldbruck, Germany). As solid phase a column with spherical, polystyrene-based cation exchanger (Peek LCA N04/Na, dimension 150 x 4.6 mm) from SYKAM was used. Depending on the L-amino acid the separation takes place in an isocratic run using a mixture of buffers A and B for elution or by gradient elution using said buffers. As buffer A an aqueous solution containing in 20 I 263 g trisodium citrate, 120 g citric acid, 1100 ml methanol, 100 ml 37 % HCl and 2 ml octanoic acid (final pH 3.5) was used. As buffer B an aqueous solution containing in 20 I 392 g trisodium citrate, 100 g boric acid and 2 ml octanoic acid (final pH 10.2) was used. The free amino acids were coloured with ninhydrin through post-column derivatization and detected photometrically at 570 nm.

### 11. Glucose determination with continuous flow system (CFS)

A SANplus multi-channel continuous flow analyser from SKALAR analytic GmbH (Erkelenz, Germany) was used to determine the concentration of glucose in the supernatant. Glucose was detected with a coupled-enzyme assay (Hexokinase/ Glucose-6-Phosphate-Dehydrogenase) via NADH formation.

### B) EXPERIMENTAL RESULTS

### Example 1

Sequence of the *pntA and pntB* genes derived from *Escherichia coli* and *pntA and pntB* genes derived from *Corynebacterium urealyticum*

The *pntA* (SEQ ID NO: 1) and *pntB* (SEQ ID NO: 2) genes derived from *Escherichia coli* code for a pyridine nucleotide transhydrogenase subunit alpha and pyridine nucleotide transhydrogenase subunit beta, respectively. The encoded polypeptide sequence can be found at the NCBI under NCBI Reference Sequence: *pntA:* NP_416120.1 (SEQ ID NO: 3), *pntB:* NP_416119.1 (SEQ ID NO: 4).

The *pntA* (NCBI Reference Sequence: CKV82_RS04765) (SEQ ID NO: 5) *and pntB* (NCBI Reference Sequence: CKV82_RS04770) (SEQ ID NO: 6) genes derived from *Corynebacterium urealyticum* code for a NAD(P)(+) transhydrogenase subunit alpha and NAD(P)(+) transhydrogenase subunit beta, respectively. The encoded polypeptide sequence can be found at the NCBI under the description Re/Si-specific NAD(P)(+) transhydrogenase subunit alpha as NCBI Reference Sequence: *pntA:* WP_012360758.1 (SEQ ID NO: 7) and *pntB:* WP_012360759.1 (SEQ ID NO: 8) under the description NAD(P)(+) transhydrogenase (Re/Si-specific) subunit beta.

### Example 2

### Construction of plasmids pK18mobsacB_pntAB_ec and pK18mobsacB_pntAB_cu

Plasmids pK18mobsacB_pntAB_ec and pK18mobsacB_pntAB_cu were constructed to enable incorporation of the heterologous *pntAB* genes *pntAB_ec* and *pntAB_cu* in lysine producing strains DM1547.1. The plasmid is based on the mobilizable vector pK18mobsacB described by Schäfer et al. (Gene 145, 69-73, 1994). For construction of pK18mobsacB_*pntAB*_ec and pK18mobsacB_*pntAB*_cu, the *pntAB_ec* polynucleotide according to SEQ ID NO: 9 (carrying intergenic sequences, promotor sj, pntA gene and pntB gene derived from *Escherichia coli)* and the *pntAB_cu* polynucleotide according to SEQ ID NO: 10 (carrying intergenic sequences, promotor sj, *pntA* gene and *pntB* gene derived from *Corynebacterium urealyticum)* were synthesized and subcloned into pK18mobsacB by GeneArt (ThermoFisher Scientific (Waltham, USA)).

The intergenic sequences of SEQ ID: 9 and SEQ ID: 10 derived from *Corynebacterium glutamicum* represent homologous sequences which enable to incorporate the heterologous *pntA* and *pntB* genes by homologous recombination.

Seq ID NO: 9 represents the codon optimized variant of *pntA* and *pntB* gene sequences which were used for construction the plasmid pK18mobsacB_*pntAB*_ec.

Seq ID NO: 10 comprises an ATG nucleotide as start codon which is a variant of the native *pntA* gene sequence derived from *Corynebacterium urealyticum* which was used for construction the plasmid pK18mobsacB_*pntAB*_cu.

To assemble the plasmids pK18mobsacB_pntAB_ec the following steps were done by GeneArt: The two polynucleotides i.e., the vector pK18mobsacB and the polynucleotide *pntAB_ec* were treated with Xmal and Sbfl, ligated and the ligation mixture used to transform *E. coli.*

To assemble the plasmids pK18mobsacB_*pntAB*_cu the following steps were done by GeneArt: The two polynucleotides i.e., the vector pK18mobsacB and the polynucleotide *pntAB_cu* were treated with Xbal and Sbfl, ligated and the ligation mixture used to transform *E. coli.*

DNA of plasmids pK18mobsacB_*pntAB*_ec and pK18mobsacB_*pntAB*_cu was isolated from a transformant and the polynucleotides *pntAB_ec* and *pntAB_cu,* respectively, created within pK18mobsacB was analyzed by Sanger sequencing.

### Example 3

### Construction of strains DM1547.1_pntAB_ec and DM1547.1_pntAB_cu

Strain DM1547 is an L-lysine producer which was prepared by multiple, non-directed mutagenesis, selection, and mutant choice from *C. glutamicum* wild type strain ATCC13032. The strain is resistant to the lysine analogue S-(2-aminoethyl)-L-cysteine.

A pure culture of the DM1547 strain has been deposited with the Deutsche Sammlung für Mikroorganismen and Zellkulturen (DSMZ, Braunschweig, Germany) as DSM 13994 in accordance with the Budapest Treaty on 16 Jan. 2001. The strain DSM 13994 was described in EP1239040A2.

DM1547.1 is a successor of DM1547 which has increased activity of known beneficial lysine production genes *asd, aspB, dapA, dapB, lysA, lysC* (feedback resistant) as described in US 9,109,242 B2 and *ddh* as described in EP 4 202 046 A1 to obtain a L-lysine yield ≥ 50% (g/g) (substrate specific product yields as g _{L-lysine} HCl per g _{glucose}).

The activity of these genes was increased by use of a stronger promotor and/or introduction of additional copies or variants of the respective gene to allow for unlimited flux through the lysine biosynthesis pathway.

The plasmids pK18mobsacB_*pntAB*_ec and pK18mobsacB_*pntAB*_cu obtained in example 2 were used to incorporate the heterologous genes *pntAB_*ec derived from *E. coli* and *pntAB_cu* derived from *C. urealyticum,* respectively.

Chemically competent cells of *E. coli* strain S17-1 were transformed with plasmid DNA of pK18mobsacB_*pntAB*_ec and pK18mobsacB_*pntAB*_cu. The presence of the plasmids obtained in example 2 were analyzed by Sanger sequencing, respectively.

For conjugal transfer into the strain DM1547.1 the modified conjugation method of Schäfer et al. (Journal of Bacteriology 172, 1663 - 1666, 1990) as described in materials and methods was used. Subsequently selection of transconjugant clones was done by identification of saccharose resistance and kanamycin sensitivity phenotype.

Transconjugant clones of DM1547.1 carrying the heterologous *pntAB* genes *pntAB_ec* or *pntAB_cu* were selected according to chromosome sequence analysis. The strains carrying the heterologous *pntAB*_ec gene was called and DM1547.1_*pntAB*_ec.

The strain carrying the heterologous *pntAB_cu* gene was called DM1547.1_*pntAB*_cu.

A glycerol stock culture of the transconjugant clone was prepared and used as starting material for further investigations.

### Example 4

### L-lysine production

Strains DM1547.1 (reference), DM1547.1_*pntAB*_ec carrying the *pntAB_ec* genes derived from *E. coli and* strain DM1547.1_*pntAB_*cu carrying the *pntAB_cu* genes derived from *C. urealyticum* were analyzed for their ability to produce L-lysine from glucose by batch cultivation using the cultivation system according to Wouter Duetz.

As medium SK medium containing 20 g/l glucose as carbon source was used. The cultures were incubated for 45 h until complete consumption of glucose as confirmed by glucose analysis using blood glucose-meter and the concentrations of L-lysine and the optical density OD660 were determined. The result of the experiment is presented in table 4.

**Table 4: L-lysine production.**

| strain | OD660 | L-lysine¹ (g/l) | Y_{PS}² (g/g) |
|---|---|---|---|
| DM1547.1 | 1,36 | 11,25 | 0,56 |
| DM1547.1_*pntAB*_ec | 1,26 | 12,02 | 0,60 |
| DM1547.1_*pntAB*_cu | 1,02 | 13,44 | 0,67 |

| | | | |
|---|---|---|---|
| ¹ as L-lysine x HCl ² substrate specific product yields as g _{L-lysine} per g _{glucose} | | | |

Data represent mean values of eight independent cultivations.

The experiment shows that expression of heterologous *pntAB_cu* genes in strain DM1547.1_*pntAB*_cu result in a strongly increased L-lysine production as compared to the parental strains DM1547.1 and to DM1547.1*_pntAB*_ec, respectively.

This strong increase has not been seen by analyzing strain DM1547.1_*pntAB*_ec which realize the already described expression of heterologous *pntAB_ec* genes derived from *E*. *coli.*

## Claims

1. A *C. glutamicum* strain comprising overexpressed genes coding for enzymes having the function of aspartate-semialdehyde dehydrogenase, aspartate aminotransferase, dihydrodipicolinate synthase, dihydrodipicolinate reductase, diaminopimelate decarboxylase, aspartatokinase and diaminopimelate dehydrogenase and comprising a gene coding for the NAD(P)(+) transhydrogenase subunit alpha PntA comprising an amino acid sequence with an identity of at least 80 % to the amino acid sequences according to SEQ ID NO: 7 and a gene coding for the NAD(P)(+) transhydrogenase subunit beta PntB comprising an amino acid sequence with an identity of at least 80 % to the amino acid sequences according to SEQ ID NO: 8.

2. The *C. glutamicum strain* of claim 1, further comprising at least one copy of a gene *lysC* coding for a feedback resistant aspartokinase polypeptide variant.

3. The *C. glutamicum strain* of claim 2, wherein the amino acid sequence of the feedback resistant aspartokinase polypeptide variant comprises SEQ ID NO: 12.

4. The *C. glutamicum* strain of any of the preceding claims comprising in its chromosome a polynucleotide encoding an amino acid sequence of a NAD(P)(+) transhydrogenase subunit alpha PntA comprising an amino acid sequence according to SEQ ID NO: 7 and a polynucleotide encoding an amino acid sequence of a NAD(P)(+) transhydrogenase subunit beta PntB comprising an amino acid sequences according to SEQ ID NO: 8.

5. A method for the fermentative production of an L-lysine comprising the steps of cultivating the *C. glutamicum* strain of any of the preceding claims and accumulating L-lysine in the medium to form an L-lysine containing fermentation broth.

6. The method of claim 5, further comprising manufacturing an L-lysine containing product from said fermentation broth.

7. The method of claim 6, further comprising isolating L-lysine from the L-lysine containing fermentation broth.
